# EUROPEAN PATENT APPLICATION

(11) **EP 1 510 517 A1**
(43) Date of publication of application: **02.03.2005**
(21) Application number: 03077734.6
(22) Date of filing: 01.09.2003
(51) Int. Cl.: C07D 333/20, C07D 307/52

(54) **Process for the asymmetric hydrogenation of beta-amino ketones**

(71) Applicant: Lonza AG, 4052 Basel (CH)
(72) Inventor: The designation of the inventor has not yet been filed

(57) **Abstract**

The invention relates to a process for the preparation of enantiomerically enriched or enantiomerically pure (*S*)- or (*R*)-*N*-monosubstituted β-amino alcohols of formula 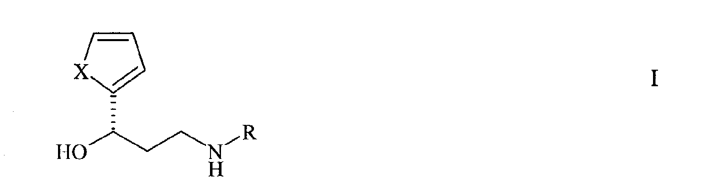 and enantiomers
and their addition salts of proton acids, X represents S or O, and R represent C₁₋₆-alkyl, C₃₋₈-cycloalkyl, aryl or aralkyl.

## Description

The invention relates to a process for the preparation of enantiomerically enriched or enantiomerically pure (*S*)- or (*R*)-*N*-monosubstituted β-amino alcohols of formula 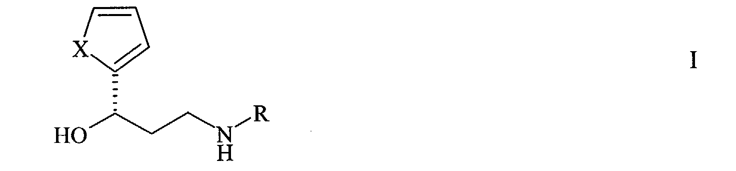 and enantiomers
and their addition salts of proton acids which can be obtained by asymmetric hydrogenation of β-amino ketones of formula 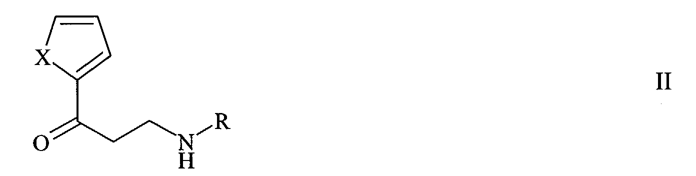 and their addition salts of proton acids.

*N*-Monosubstituted β-amino alcohols of formula I like (*S*)-(-)-3-*N*-methylamino-1-(2-thienyl)-1-propanol (I (X = S, R = methyl)) are useful key intermediates and building blocks for the preparation of pharmaceutically active compounds like (*S*)-(+)-methyl-[3-(1-naphthyloxy)-3-(2-thienyl)-propyl]-amine ((*S*)-duloxetine, see e.g. Liu, H. et al., *Chirality* **2000,** *12*, 26-29), which acts as neuro-active compound strongly inhibiting the serotonine and norephedrine uptake (Deeter, J. et al., *Tetrahedron Lett.* **1990**, *31*, 7101-7104).

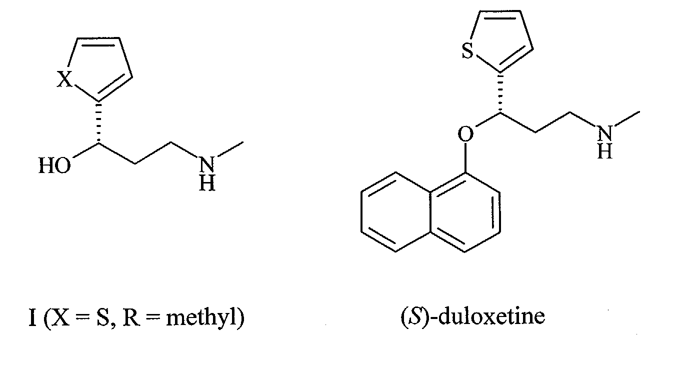

EP-A-457559 and EP-A-650965 disclose the preparation of *N,N-*dimethyl β-amino alcohols via Mannich-type reactions of methyl ketones with paraformaldehyde and dimethylamine followed by reduction of the carbonyl group. After reaction of the hydroxyl group affording an alkyl or aryl ether derivative, one *N*-methyl radical is removed to obtain *N*-monosubstituted compounds.

In WO-A-03/062219, JP-A-2003-192681 and Sorbera L. A. et al. *(Drug Future* **2000,** 25, 907-916) several strategies for syntheses of duloxetine are presented, which were published during the last years. A common synthesis principle in processes mentioned therein is the formation of the hydroxy group via hydrogenation of a carbonyl group. There are two main strategies for the preparation of the chiral alcohols or derivatives thereof.
The first one utilizes chiral resolution of racemic alcohols resulting from achiral hydrogenation of the corresponding *N,N* dialkyl-β-amino ketones.
The second route is asymmetric hydrogenation of *N,N*-dialkyl-β-amino ketones using either chiral metal-ligand complexes or achiral complexes together with optically active auxiliaries as hydrogenation catalysts.
A common feature is the consequent use of amino-protective groups which were removed as one of the last steps before the final active compound is obtained. Preferably, methyl and benzyl groups were used to protect the amino group.

In EP-A-1254885, asymmetric hydrogenation of several *N*,*N*-disubstituted α-, β- and γ-amino ketones in the presence of catalyst systems consisting of ruthenium-phosphine-complexes is disclosed. The catalyst system comprises bidentate amino and bidentate phosphine ligands complexing the Ruthenium (Ru) ion.
Asymmetric hydrogenation of said amino ketones containing at least one -CH₂-NR²R³ group wherein R² is acyl or alkoxycarbonyl and wherein R³ is hydrogen is disclosed on page 4, line 14ff. The particular combinations wherein R³ is hydrogen and wherein R² is alkyl, cycloalkyl, aryl or aralkyl are not disclosed.

In a known process for the preparation of an optically active precursor of (S)-duloxetine, a catalyst system for asymmetric hydrogenation of 3-*N*-dimethylamino-1-(2-thienyl)-1-propanone is disclosed in Ohkuma, T. et al. *(Org. Lett.* **2000**, 2, 1749-1751). In spite of several examples for *N,N-*di substituted β-amino ketones, asymmetric hydrogenation of *N*-monosubstituted β-amino ketones is not disclosed.

Sakuraba S. et al. (*Chem. Pharm. Bull.* **1995**, *43,* 748-753) discloses direct asymmetric hydrogenation of 3-*N*-methylamino-1-phenyl-1-propanone resulting in an enantiomeric excess of < 80 %. A process for asymmetric hydrogenation and subsequent debenzylation of amino ketones containing a benzyl protective group is the main feature, presented in scheme 2. Hydrogenation of amino ketones containing heteroaromatic residues is not disclosed.

A further process for both selective and asymmetric hydrogenation of amino ketones is disclosed in WO-A-02/055477. A central nitrogen atom forms a α- and a β-amino ketone moiety within the same molecule. The nitrogen atom is further substituted by a methyl group and no N-H group is present.

All known processes used in synthesizing precursors of pharmaceutically active compounds like (*S*)-duloxetine by hydrogenation of amino ketones are characterized in that the amino groups don't contain active hydrogen atoms.

Preparation of *N*-monosubstituted β-keto amines of formula II and asymmetric hydrogenation of the carbonyl group establishes an alternative and economically advantageous synthetic route for industrial production of optically active derivatives of *N*-monosubstituted β-amino alcohols of formula I, like (*S*)-duloxetine.

Racemic mixtures of the enantiomers of compounds of formula I, can be prepared according to the method described in International Application No. PCT/EP03/07411. Presently, the main drawback is that the corresponding alcohols are available as racemic mixtures only. No method is disclosed for an efficient enantioselective reduction process of *N*-monosubstituted β-keto amines.

In the following the terms "β-amino ketones" and "β-amino alcohols", more specifically "(*S*)-*N*-monosubstituted β-amino alcohols", include the pure compounds and their physiologically acceptable addition salts of proton acids.

The term "enantiomerically enriched compound" comprises optically active compounds with an enantiomeric excess (ee) of at least 70 %.

The term "enantiomerically pure compound" comprises optically active compounds with an enantiomeric excess of at least 90 %.

The term "C₁₋₆-alkyl" represents a linear or branched alkyl group having 1 to 6 carbon atoms, for example methyl, ethyl, propyl, isopropyl, *n*-butyl, isobutyl, *sec*-butyl, *tert*-butyl, pentyl and hexyl.

The term "C₃₋₈-cycloalkyl" represents a cycloaliphatic group having 3 to 8 carbon atoms, i.e. cyclopropyl, cyclobutyl, cyclopentyl, cyclohexyl, cycloheptyl and cyclooctyl.

The term "aryl" represents an optionally substituted aromatic group, preferably phenyl or naphthyl, wherein the substituents optionally being further substituted with one or more C₁₋₄-alkyl groups and/or halogen atoms.

The term "aralkyl" represents an optionally further substituted aryl moiety consisting of phenyl or naphthyl bound to the molecule in question via a linear C₁₋₄-alkyl moiety which can be further substituted by halogen atoms. The substituents of the aryl moiety can be one or more C₁₋₄-alkyl groups and/or halogen atoms.

The technical problem to be solved by the present invention was to provide a selective and high-yield process for the asymmetric hydrogenation of *N*-monosubstituted β-ketoamines to get enantiomerically enriched or enantiomerically pure (*S*)- or (*R*)-*N*-monosubstituted β-amino alcohols without using protective groups for the secondary amino group.

Another target of the present invention was to provide *N*-monosubstituted β-amino alcohols.

The problems mentioned above could be solved according to the process of claim 1.

The present invention provides a process for the preparation of chiral compounds of formula 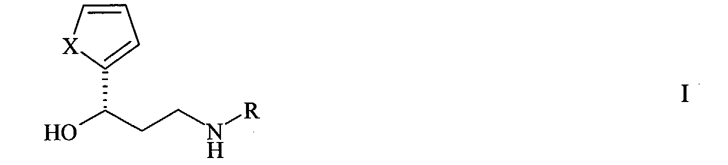 and enantiomers
wherein X represents S or O, and R represents C₁₋₆-alkyl, C₃₋₈-cycloalkyl, aryl or aralkyl, each aryl or aralkyl being optionally further substituted with one or more C₁₋₄-alkyl groups and/or halogen atoms,
which process comprises the asymmetric hydrogenation of compounds of formula 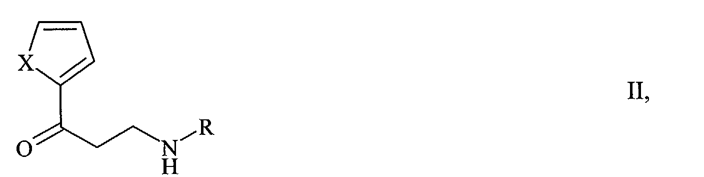 wherein X and R are as defined above,
in the presence of a transition metal complex of a chiral bidentate phosphine ligand and, optionally, a base.

In a preferred embodiment the chiral bidentate phosphine ligand is a compound of formula 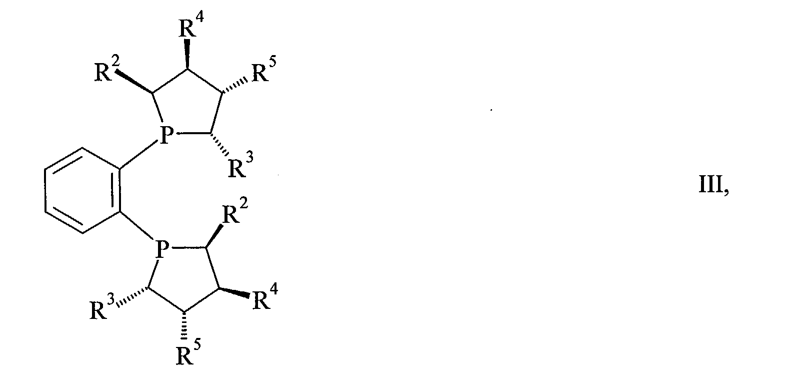 and enantiomers
wherein R² and R³ are methyl, ethyl or isopropyl; and whereinR⁴ and R⁵ are hydrogen or R⁴ and R⁵ together form a isopropylidenedioxy group.
Particularly preferred R² and R³ are methyl, ethyl or isopropyl and R⁴ and R⁵ are hydrogen; or R² and R³ are methyl and R⁴ and R⁵ together form a isopropylidenedioxy group.

Ligands of the family of DuPhos-ligands of formula III wherein R³ is methyl, ethyl or isopropyl and wherein R⁴ and R⁵ are hydrogen are sold by Chirotech Technology Ltd.

Ligands of the family of KetalPhos-ligands of formula III wherein R³ is methyl and wherein R⁴ and R⁵ together form a isopropylidenedioxy group are available from Chiral Quest, Inc.

Preferably the transition metal is Ruthenium (Ru) or Rhodium (Rh). Particularly preferred the transition metal is Rh.

In a further preferred embodiment the chiral bidentate phosphine ligand is a compound of formula 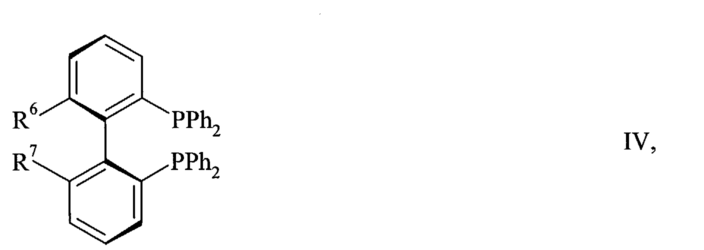 and enantiomers
wherein R⁶ and R⁷ are methoxy or ethoxy or wherein R⁶ and R⁷ together form a 1,3-propylidenedioxy or a 1,4-butylidenedioxy group.

Particularly preferred the chiral bidentate phosphine ligand is selected from the group consisting of *(S, S)-* or (R, R)-Me-DuPhos, *(S, S)-* or (R, R)-Et-DuPhos, *(S,S,S,S)-* or (*R*,*R*,*R*,*R*)-Me-KetalPhos, (S)- or (*R*)-C4-TunaPhos and (S)- or (*R*)-MeOBiPhep. More particularly preferred the chiral bidentate phosphine ligand is selected from the group consisting of (*S*,*S*)-Me-DuPhos, (*S*,*S*)-Et-DuPhos, (*S*,*S*,*S*,*S*)-Me-KetalPhos, (*S*)-C4-TunaPhos and (*S*)-MeOBiPhep of the following formulae

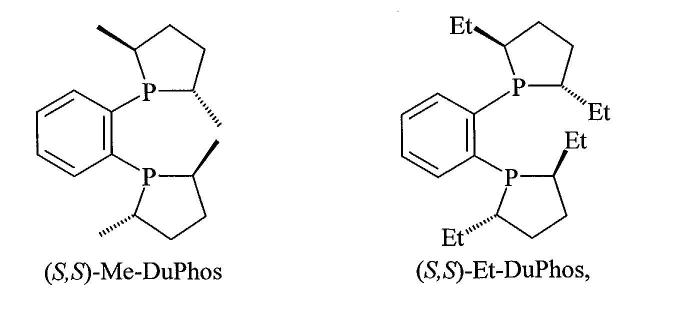

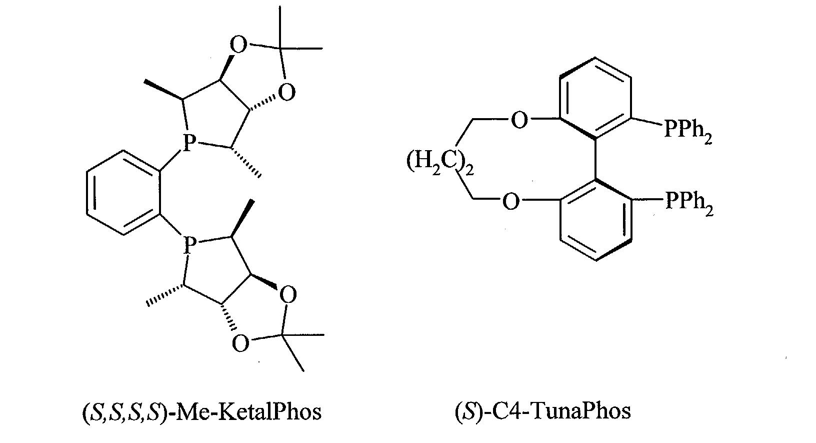

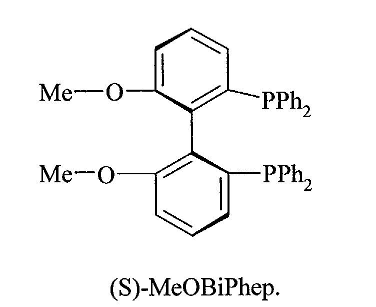

The catalyst precursor complex optionally comprises at least one further stabilizing ligand such as a diene, alkene or arene. In a preferred embodiment the stabilizing ligand is 1,5-cyclooctadiene (cod) or *p*-cymene (cym). Particularly preferred the stabilizing ligand is 1,5-cyclooctadiene.

The hydrogenation is carried out with a catalyst solution in a polar solvent. Preferably the polar solvent is methanol, ethanol or isopropyl alcohol or a mixture thereof. The solution may contain further additives like ethyl acetoacetate (AAEt).

The catalyst solution can be prepared in situ by dissolving a transition metal salt MY, where M is Ru or Rh and where Y is Cl⁻, BF₄⁻, AsF₆⁻, SbF₆- or OTf⁻ (trifluormethansulfonate or triflate), or another suitable counterion, in a polar solvent and mixing with a suitable amount of the chiral ligand, optionally further mixed with the stabilizing ligand. Alternatively, the catalyst solution can be obtained by mixing a transition metal complex which already contains a stabilizing ligand with a suitable amount of the chiral ligand. Furthermore, the catalyst solution can be obtained by dissolving a preformed chiral transition metal-ligand complex which already contains further stabilizing ligands.

In a preferred embodiment the catalyst precursor complex is prepared by mixing a transition metal complex of the formulae [Rh(cod)₂]⁺ BF₄⁻ or [Ru₂Cl₄(cym)_{2]} with a chiral bidentate phosphine selected from the group consisting of Me-DuPhos, Et-DuPhos and Me-KetalPhos . More preferably the chiral bidentate phosphine is selected from the group consisting of (*S*,*S*)-Me-DuPhos, (*S*,*S*)-Et-DuPhos and (*S*,*S*,*S*,*S*)-Me-KetalPhos.
Preferably the metal salt MY or the transition metal complex is mixed with the chiral bidentate phosphine at a ratio of 1:5 to 5:1. More preferably the precursor/phosphine ratio is in the range of 1:2 to 2:1. Most preferably the precursor/phosphine ratio is 1:1.

In a particular embodiment the counterion of the transition metal salt, the catalytic precursor complex and the transition metal complex of a chiral bidentate phosphine ligand is Cl⁻ or BF₄⁻.

Optionally the hydrogenation solution may contain a base to facilitate forming of the substrate-catalyst complex and to neutralize acids which may be part of the starting compounds. In a preferred embodiment the base is a hydroxide, methanolate or ethanolate of lithium, sodium or potassium or a mixture of said bases.
Preferably the base added is in an amount of 0.6 to 1.2 eq to the amount of the starting compounds. More preferably the amount of the base added is in the range of 0.7 to 1.0 eq. The base can be added to the catalyst solution before, during or after the addition of the starting compounds. It can be added at once, in a continuous manner or in separate portions.

In a preferred embodiment the hydrogen pressure during the reaction is in the range of 1 to 60 bar and more particularly preferred in the range of 10 to 30 bar.

The hydrogenation can be carried out at a temperature in the range of 20 to 80 °C. Preferably the temperature is in the range of 30 to 50 °C.

The present invention also provides compounds of formula 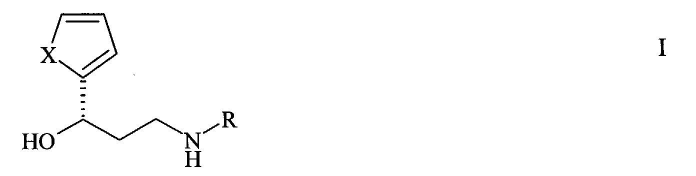 and their addition salts of proton acids, wherein X is S or O and R represents C₁₋₆-alkyl, C₃₋₈-cycloalkyl, benzyl with the exception of a compound wherein X is S and R is methyl. In a preferred embodiment benzyl can be independently further substituted with C₁₋₄-alkyl or halogen atoms.

The present invention is illustrated by the following non-limiting examples.

### General Procedure for Examples 1 to 6:

A mixture of methyl ketone, primary alkylamine and/or an addition salt thereof (1.1 to 1.5 equivalents (eq)), formaldehyde (1.4 to 1.5 eq), a solvent, optionally in the presence of a proton acid, is heated in an autoclave at a total pressure above 1.5 bar for 5 to 24 hours. Afterwards, the reaction solution is cooled to 20 °C. Optionally the reaction solvent can than be removed partly or in whole and a solvent like ethyl acetate or isopropanol can be added under vigorous stirring, if necessary to facilitate precipitation of the product. The suspension is cooled (0 to 20 °C) and after precipitation (0.5 to 10 hours) the product can be filtrated, optionally washed and dried affording a slightly yellow to white powder in yields between 50 to 75 %. The product can be recrystallized from isopropanol and/or ethyl acetate if necessary. If the stability of the free base is sufficient at ambient conditions, extracting with an organic solvent and an aqueous base affords the free base.

### Example 1:

### 3-(Methylamino)-1-(thiophen-2-yl)propan-1-one · HCl (II, X = S, R = methyl)

2-Acetylthiophene (25.5 g, 200 mmol); methylamine hydrochloride (14.9 g, 220 mmol, 1.1 eq); paraformaldehyde (8.2 g, 280 mmol, 1.4 eq); HCl conc. (1.0 g); ethanol (100 mL); 110 °C for 9 hours; ca. 2 to 2.5 bar; removing of ethanol (50 mL) *in vacuo;* addition of ethyl acetate (200 mL); ca. 71 % yield.
¹H-NMR δ (DMSO-d₆, 400 MHz): 9.16 (2 H, s, br), 8.07 (1 H, dd, *J*= 5.0, 1.0), 8.01 (1 H, dd, *J*= 3.8, 1.0), 7.29 (1 H, dd, *J*= 5.0, 3.8), 3.49 (2 H, t), 3.20 (2 H, t), 2.56 (3 H, s); ¹³C-NMR δ (DMSO-d₆, 100 MHz): 189.9, 142.7, 135.4, 133.8, 128.8, 43.1, 34.6, 32.4.

### Example 2:

### 3-(Methylamino)-1-(thiophen-2-yl)propan-1-one · HCl (II, X = S, R = methyl)

2-Acetylthiophene (24.9 g, 197 mmol); methylamine hydrochloride (14.8 g, 219 mmol, 1.1 eq); paraformaldehyde (8.3 g, 276 mmol, 1.4 eq); HCl conc. (1.1 g); isopropanol (100 mL); 110 °C for 8 hours; ca. 2 to 2.5 bar; addition of isopropanol (50 mL); ca. 65 % yield.

### Example 3:

### 3-(Ethylamino)-1-(thiophen-2-yl)propan-1-one · HCl (II, X = S, R = ethyl)

2-Acetylthiophene (6.3 g, 50 mmol); ethylamine hydrochloride (6.1 g, 75 mmol, 1.5 eq); paraformaldehyde (2.1 g, 75 mmol, 1.5 eq); HCl conc. (0.3 g); ethanol (35 mL); 110 °C for 9 hours; ca. 2 to 2.5 bar; removing of ethanol (25 mL) *in vacuo;* addition of ethyl acetate (50 mL); ca. 73 % yield.
¹H-NMR δ (DMSO-d₆, 400 MHz) : 9.3 (2 H, s, br), 8.08 (1 H, dd), 8.00 (1 H, dd), 7.28 (1 H, dd), 3.51 (2 H, t), 3.20 (2 H, t), 2.96 (2 H, q), 1.23 (3 H, t).

### Example 4:

### 3-(Isobutylamino)-1-(thiophen-2-yl)propan-1-one · HCl (II, X = S, R = isobutyl)

2-Acetylthiophene (6.3 g, 50 mmol); isobutylamine hydrochloride (8.3 g, 75 mmol, 1.5 eq); paraformaldehyde (2.1 g, 75 mmol, 1.5 eq); HCl conc. (0.3 g); ethanol (35 mL); 110 °C for 9 hours; ca. 2 to 2.5 bar; removing of ethanol (35 mL) *in vacuo;* addition of ethyl acetate (50 mL); ca. 56 % yield.
¹H-NMR δ (DMSO-d₆, 400 MHz) : 9.0 (2 H, s, br), 8.08 (1 H, dd), 7.99 (1 H, dd), 7.29 (1 H, dd), 3.55 (2 H, t), 3.22 (2 H, t), 2.78 (2 H, d), 2.03 (1 H, m), 0.96 (6 H, d).

### Example 5:

### 3-(tert- Butylamino)-1-(thiophen-2-yl)propan-1-one · HCl (II, X = S, R = tert-butyl)

2-Acetylthiophene (6.3 g, 50 mmol); *tert*-butylamine hydrochloride (8.3 g, 75 mmol, 1.5 eq); paraformaldehyde (2.1 g, 75 mmol, 1.5 eq); HCl conc. (0.3 g); butanol (35 mL); 117 °C for 9 hours; ca. 2 to 2.5 bar; addition of ethyl acetate (50 mL); ca. 52 % yield.
¹H-NMR δ (DMSO-d₆, 400 MHz) : 9.2 (2 H, s, br), 8.08 (1 H, dd), 7.98 (1 H, dd), 7.30 (1 H, dd), 3.54 (2 H, t), 3.19 (2 H, t), 1.34 (9 H, s).

### Example 6:

### 3-(Methylamino)-1-(furan-2-yl)propan-1-one · HCl (II, X = O, R = methyl)

2-Acetylfuran (7.5 g, 68 mmol); methylamine hydrochloride (6.9 g, 102 mmol, 1.5 eq); paraformaldehyde (3.1 g, 102 mmol, 1.5 eq); HCl conc. (1.15 g); ethanol (35 mL); 110 °C for 8 hours; ca. 2 to 2.5 bar; removing of ethanol (30 mL) *in vacuo;* addition of ethyl acetate (50 mL); ca. 64 % yield.
¹H-NMR δ (DMSO-d₆, 400 MHz) : 9.0 (2 H, s, br), 8.05 (1 H, m), 7.53 (1 H, m), 6.77 (1 H, m), 3.34 (2 H, t), 3.2 (2 H, m), 2.57 (3 H, s, br).

### General Procedure for Examples 7 to 16:

### (S)-3-N-Methylamino-1-(2-thienyl)-1-propanol (I, X = S, R = methyl)

Hydrogenations were run in a Chemscan-Screening equipment (10 mL scale, up to 8 reactions per time) testing combinations of catalyst precursors (0.02 mmol) with chiral phosphine ligands (0.02 mmol). All reactions were run with 0.4g 3-(methylamino)-1-(thiophen-2-yl)propan-1-one · HCl (II, X = S, R² = methyl) 90% in 6 mL MeOH at 30 bar H₂ for at least 24 h using a substrate to catalyst ratio (S/C ratio) of 100. Because of the HCl content in the starting compound 0.36 g of 24% (approx 0.8 eq of the ketone) or 30% NaOMe (approx 1.0 eq of the ketone) solutions were added. The reactions were run at temperatures between 30 and 70° C. The products were analyzed by HPLC area-% for conversion and by HPLC for ee. Conditions and results are summarized in table 1 below.

### Example 17: (S)-3-N-methylamino-1-(2-thienyl)-1-propanol (I, X = S, R = methyl)

In order to confirm the excellent analytical results of example 13, this experiment was scaled up to a 50 mL autoclave with following conditions. 22 mg (S,S)-Me-DuPhos (0.07 mmol) and 28 mg [Rh(cod)₂]⁺ BF₄⁻ (0.07 mmol) were dissolved under argon in 12 mL degassed methanol and added to a solution of 1.60 g 3-(methylamino)-1-(thiophen-2-yl)propan-1-one · HCl (7.8 mmol) in 12 mL methanol in a stainless steel autoclave. To this solution were added 1.44 g NaOMe 24% (6.4 mmol). The substrate was hydrogenated at 30-34°C and 30 bar hydrogen for 5 h. The product solution was filtered, the filtrate evaporated and the residue dissolved in MTBE/water. The organic phase was evaporated to give 0.89 g (S)-3-N-methylamino-1-(2-thienyl)-1-propanol (5.2 mmol, 67% yield). According to chiral HPLC ee of the product is > 99%.

**Table 1:**

| No. | metal complex | ligand | additive | T [°C] | ee [%] | yield [%] |
|---|---|---|---|---|---|---|
| 7 | Rh(cod)₂BF₄ | (*S*)-MeOBiPhep | 0.36 g A | 50 | 67 | 12.7 |
| 8 | Rh(cod)₂BF₄ | (*S*)-MeOBiPhep | 0.36 g B | 30 | 81 | 20.4 |
| 9 | Rh(cod)₂BF₄ | (*S*)-MeOBiPhep | 0.36 g B 0.23 g C | 30 | >99 | 36.9 |
| 10 | Rh(cod)₂BF₄ | (*S*)-C4-TunaPhos | --- | 50 | 68 | 4.0 |
| 11 | Ru₂Cl₄(cym)₂ | (*S*)-C4-TunaPhos | --- | 50 | -40 | 11.6 |
| 12 | Rh(cod)₂BF₄ | (*S*)-C4-TunaPhos | 0.36 g A 0.23 g C | 50 | 54 | 6.2 |
| 13 | Rh(cod)₂BF₄ | (*S*,*S*)-Me-DuPhos | 0.36 g A | 50 | >99 | 15.2 |
| 14 | Rh(cod)₂BF₄ | (*S*,*S*)-Me-DuPhos | 0.36 g B | 30 | >99 | 71.3 |
| 15 | Rh(cod)₂BF₄ | (*S*,*S*)-Me-DuPhos | 0.36 g B 0.23 g C | 30 | >99 | 30.5 |
| 16 | Rh(cod)₂BF₄ | (*S*,*S*,*S*,*S*)-Me-KetalPhos | 0.36 g B | 30 | >99 | 79.8 |
| 17 | Rh(cod)₂BF₄ | (*S*,*S*)-Me-DuPhos | 1.44 g B | 30-34 | >99 | 67.0 |
| Additives: A=NaOMe 30%, B=NaOMe 24%, C=AAEt Positive ee denotes excess of the (*S*)-enantiomer, negative ee denotes excess of the (*R*)-enantiomer | | | | | | |

## Claims

1. Process for the preparation of chiral compounds of formula 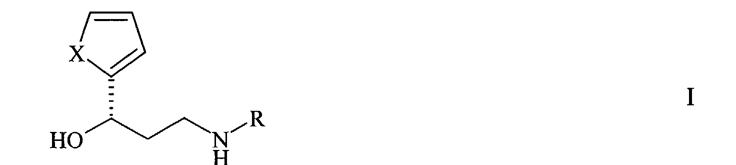 and enantiomers
wherein X represents S or O, and R represents C₁₋₆-alkyl, C₃₋₈-cycloalkyl, aryl or aralkyl, each aryl or aralkyl being optionally further substituted with one or more C₁₋₄-alkyl groups and/or halogen atoms,
which process comprises the asymmetric hydrogenation of compounds of formula 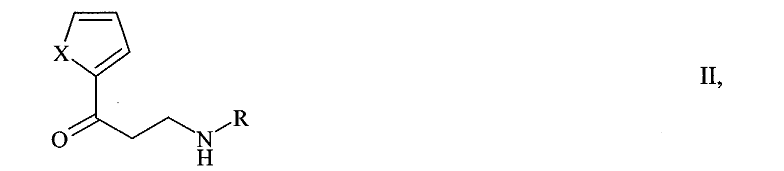 wherein X and R are as defined above,
in the presence of a transition metal complex of a chiral bidentate phosphine ligand and, optionally, a base.

2. The process of claim 1 wherein the chiral bidentate phosphine ligand is a compound of formula 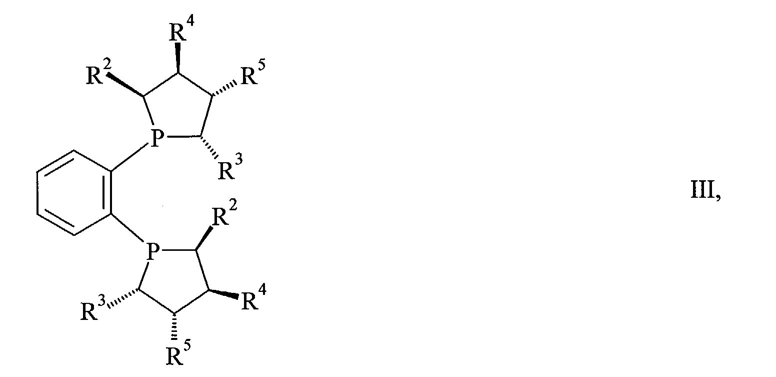 and enantiomers
wherein R² and R³ are methyl, ethyl or isopropyl; and wherein R⁴ and R⁵ are hydrogen or R⁴ and R⁵ together form a isopropylidenedioxy group.

3. The process of claim 1, wherein the chiral bidentate phosphine ligand is a compound of formula 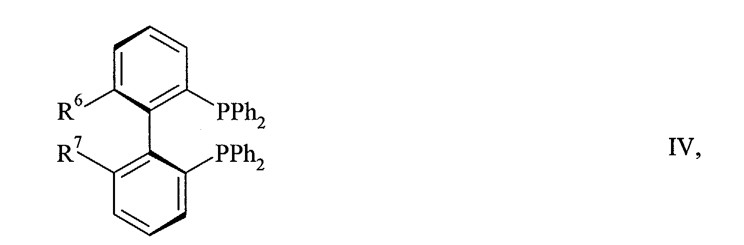 and enantiomers
wherein R⁶ and R⁷ are methoxy or ethoxy or wherein R⁶ and R⁷ together form a 1,3-propylidenedioxy or a 1,4-butylidenedioxy group.

4. The process of claim 1, wherein the chiral bidentate phosphine ligand is selected from the group consisting of (*S*,*S*)-Me-DuPhos, (*S*,*S*)-Et-DuPhos, (*S*,*S*,*S*,*S*)-Me-KetalPhos and (*S*)-C4-TunaPhos.

5. The process of any one of claims 1 to 4, wherein the transition metal is Ru or Rh.

6. The process of any one of claims 1 to 5, wherein the transition metal complex of a chiral bidentate phosphine ligand comprises at least one diene, alkene or arene as stabilizing ligand.

7. The process of claim 6, wherein the transition metal complex of a chiral bidentate phosphine ligand comprises at least one stabilizing ligand selected from the group consisting of 1,5-cyclooctadiene and *p*-cymene.

8. The process of any one of claims 1 to 7, wherein the counterion of the transition metal complex of a chiral bidentate phosphine ligand is selected from the group consisting of Cl⁻, BF₄⁻, AsF₆⁻, SbF₆⁻ and triflate.

9. The process of any one of claims 1 to 8, wherein the catalyst is prepared by mixing a transition metal complex of the formula [Rh(cod)₂]⁺BF₄⁻ with a chiral bidentate phosphine selected from the group consisting of (*S*,*S*)-Me-DuPhos, (*S*,*S*)-Et-DuPhos and (*S,S,S,S*)*-*Me-KetalPhos.

10. The process of any one of claims 1 to 9, wherein the base is a hydroxide, methanolate or ethanolate of lithium, sodium or potassium or a mixture of said bases.

11. The process of any of claims 1 to 10, wherein the hydrogen pressure during the reaction is in the range of 1 to 60 bar and more particularly preferred in the range of 10 to 30 bar.

12. Compounds of formula 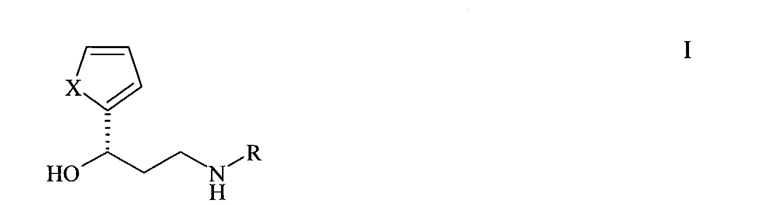 and enantiomers
and its addition salts of proton acids, wherein X represents S or O, and R represent C₁₋₆-alkyl, C₃₋₈-cycloalkyl or benzyl with the exception of compounds wherein X is S and R is methyl.
